Europäisches Patentamt

(19) European Patent Office (11) Veröffentlichungsnummer: **0 009 257**

Office européen des brevets **B1**

(12) **EUROPÄISCHE PATENTSCHRIFT**

(45) Veröffentlichungstag der Patentschrift: **19.05.82** (51) Int. Cl.³: **F 16 K 11/02, G 01 F 19/00**

(21) Anmeldenummer: **79103564.5**

(22) Anmeldetag: **21.09.79**

(54) Absperrorgan für den Auslass eines Messbehälters, insbesondere Urinmessbehälters.

(30) Priorität: **26.09.78 DE 7828577 U**

(43) Veröffentlichungstag der Anmeldung:
**02.04.80 Patentblatt 80/7**

(45) Bekanntmachung des Hinweises auf die Patenterteilung:
**19.05.82 Patentblatt 82/20**

(84) Benannte Vertragsstaaten:
**AT BE CH DE FR GB IT LU NL SE**

(56) Entgegenhaltungen:
CH - A - 559 352
DD - A - 49 097
DE - A - 2 416 034
DE - B - 1 963 451
DE - C - 56 056
DE - C - 108 084
FR - A - 2 286 326
GB - A - 1 500 424
US - A - 3 044 491
US - A - 3 416 570

(73) Patentinhaber: **Intermedicat GmbH**
**Gerliswilstrasse 45**
**CH-6020 Emmenbrücke (CH)**

(72) Erfinder: **Voges, Karl-Friedrich**
**Lindenbergstrasse 18**
**D-3508 Melsungen (DE)**
Erfinder: **Herlitze, Gerhard**
**Binsdorfer Strasse 4**
**D-3508 Baunatal 7 (DE)**

(74) Vertreter: **von Kreisler, Alek, Dipl.-Chem.**
**Deichmannhaus am Hauptbahnhof**
**D-5000 Köln 1 (DE)**

## Absperrorgan für den Auslaß eines Meßbehälters, insbesondere Urinmeßbehälters

Die Erfindung bezieht sich auf ein Absperrorgan für den Auslaß eines Meßbehälters, insbesondere Urinmeßbehälters, mit einem auf seinem Außenumfang gummielastisch nachgiebigen Dichtelement, das in einem mit Ein- und Auslaßöffnungen versehenen Gehäuse drehbar gelagert ist.

Zum Messen des Stundenurins katheterisierter Patienten werden Urinmeßgeräte, bestehend aus einem meist starren Urinmeßbehälter und einem flexiblen Urinbeutel benutzt. Um möglichst genaue Werte zur Erstellung der Flüssigkeitsbilanz zu liefern, ist der Urinmeßbehälter durch eine oder mehrere Zwischenwände in mehrere Kammern unterteilt, wodurch eine Spreizung der Skala des Urinmeßbehälters mit entsprechend verbesserter Ablesegenauigkeit erzielt wird. Das Absperrmittel für den Auslaß eines solchen Urinmeßbehälters muß dabei gewährleisten, daß die einzelnen Kammern nicht nur in bezug auf die Auslaßöffnung, sondern auch gegeneinander abgedichtet werden, damit sich in ihnen nicht nach dem Prinzip der kommunizierenden Röhren gleiches Niveau einstellt und die durch die Unterteilung des Urinmeßbehälters in einzelne Kammern erzielte Spreizung der Skala nicht aufgehoben wird.

Zur Erreichung dieses Zweckes ist bei einem bekannten Urinmeßbehälter (CH—A 559 352) jeder Kammer ein gesondertes Absperrorgan zugeordnet. Es müssen in diesem Falle mehrere Absperrorgane einzeln betätigt werden, was sowohl beim Schließen als auch beim Öffnen der einzelnen Kammern umständlich und zeitraubend ist. Die Bedienung des Urinmeßbehälters wird mit zunehmender Anzahl von Kammern schwieriger und unzuverlässiger.

Vorteilhaft wäre der Einbau eines einzigen Absperrorgans für alle Kammern, damit sich mit geringstem Bedienungsaufwand alle Kammern gleichzeitig öffnen bzw. schließen lassen, wobei gewährleistet sein muß, daß die Kammern bei geschlossenem Absperrorgan auch gegeneinander abgedichtet sind. Aus der allgemeinen Maschinenbautechnik bekannte Ventile sind als Absperrorgan für den Auslaß eines Urinmeßbehälters nicht geeignet, weil ihre Konstruktion für einen Urinmeßbehälter zu große Abmessungen und zu komplizierte Teile, bedingt bzw. nicht den gleichzeitigen Verschluß mehrerer Kammern oder ihre gleichzeitige Öffnung ermöglicht. Dies gilt auch für einen bekannten Dreiwegehahn für Benzinleitungen (GB—A—1500424), der mit einem auf seinem Außenumfang gummielastisch nachgiebigen Dichtelement arbeitet, das in einem mit zwei Einlaßöffnungen und einer Auslaßöffnung versehenen Gehäuse exzentrisch drehbar gelagert ist. Der eine Einlaß steht mit dem Haupttank in Verbindung und der andere Einlaß ist an den Reservetank angeschlossen. Das Dichtelement wirkt nur zwischen dem "Reserve"-Einlaß und dem Auslaß. Der "Haupttank"-Einlaß steht mit dem Auslaß immer in offener Verbindung und wird von der Stellung des Dichtelementes nicht beeinflußt.

Der Erfindung liegt die Aufgabe zugrunde, ein Absperrorgan für einen in mehrere Kammern unterteilten Urinmeßbehälter zu schaffen, das einen gleichzeitigen Verschluß aller Kammern gegeneinander und nach außen sowie eine gleichzeitige Öffnung aller Kammern nach außen zuläßt sowie einfach und den räumlichen Verhältnissen bei einem Urinmeßbehälter angepaßt ausgebildet ist.

Diese Aufgabe wird dadurch gelöst, daß das Gehäuse mehrere auf einer achsparallelen Linie nebeneinanderliegende Einlaßöffnungen aufweist, die jeweils mit einer von mehreren Kammern des Meßbehälters in Verbindung stehen, und daß die Dichtflächenabschnitte auf dem Dichtelement nebeneinander auf einer achsparallelen Linie angeordnet sind.

Auf diese Weise wird ein billiges, kleines Absperrorgan geschaffen, das den räumlichen Verhältnissen bei einem Meßbehälter, insbesondere Urinmeßbehälter, angepaßt ist, und das einen gleichzeitigen Verschluß aller Kammern gegeneinander und nach außen sowie eine gleichzeitige Öffnung aller Kammern nach außen zuläßt. Alle Kammern können zum sehr schnellen Entleeren des Urinmeßbehälters gleichzeitig über den vollen Querschnitt der Gehäuse-Einlaßöffnungen geöffnet werden. Außerdem lassen sich alle Kammern im wesentlichen gleichzeitig verschließen, damit nach der Entleerung sofort erneut der Füllvorgang einsetzt. Durch die Verwendung nur eines einzigen Absperrorganes für alle Kammern des Urinmeßbehälters ist eine schnelle Bedienung sowohl beim Schliessen als auch beim Öffnen der Kammern möglich. Die Handhabung des Urinmeßbehälters wird sicherer und zuverlässiger, weil ausgeschlossen ist, daß eine Kammer versehentlich nicht verschlossen oder nicht geöffnet wird. Mit geringer Betätigungskraft kann das nach dem Auf-Zu-Prinzip einfach und sicher funktionierende Absperrorgan leicht verstellt werden. Der einfache Aufbau des Absperrorganes gestattet seine dem Einmalgebrauch angemessene preiswerte Herstellung.

In vorteilhafter Ausgestaltung der Erfindung ist das Dichtelement rotationssymmetrisch gestaltet und in dem Gehäuse exzentrisch drehbar gelagert. Die Innenwand des Gehäuses weist zweckmäßigerweise im Bereich der Einlaßöffnungen zu dem Mittelpunkt des Dichtelementes einen Radius auf, der um ein Geringes kleiner als der Radius der unbelasteten Oberfläche des Dichtelementes ist. Hierdurch wird eine angemessene Verpressung des Dichtelementes in Schließstellung erzielt.

In der Zeichnung ist ein Ausführungsbeispiel der Erfindung schematisch dargestellt. Es zeigt:

Figur 1 eine Draufsicht eines in mehrere Kammern unterteilten Urinmeßbehälters mit angehängtem Urinbeutel,

Figur 2 einen Längsschnitt eines Absperrorganes,

Figur 3 einen Querschnitt des Absperrorganes nach Figur 2 in Schließstellung des Dichtelementes, und

Figur 4 einen Querschnitt des Absperrorganes nach Figur 2 in Öffnungsstellung des Dichtelementes.

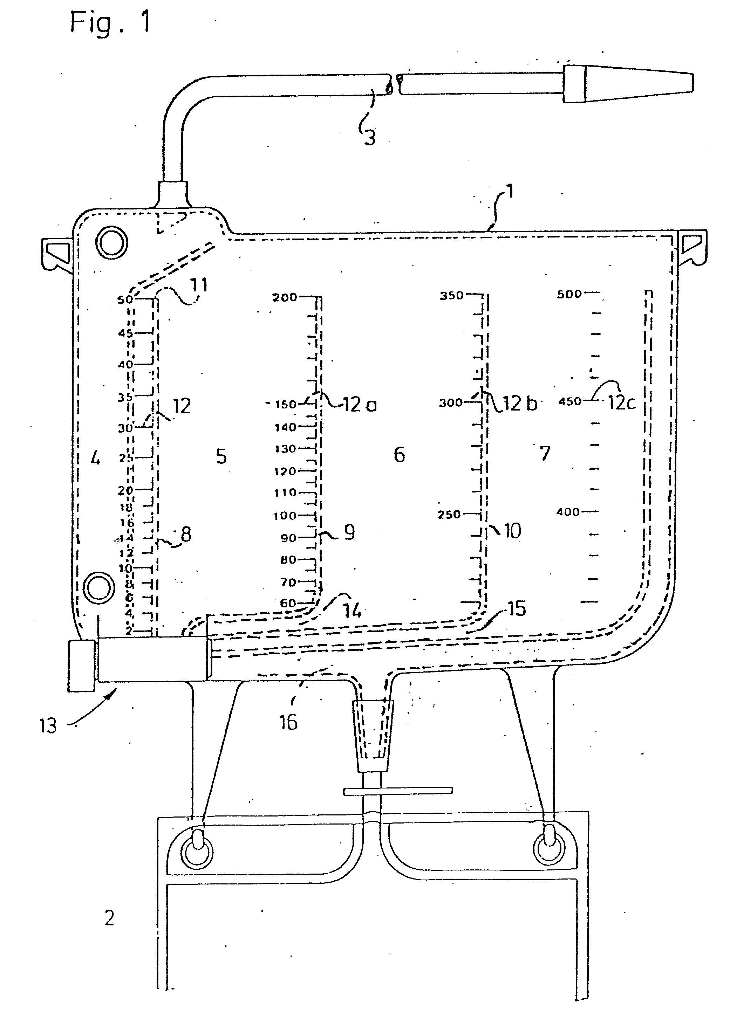

Gemäß Figur 1 besteht das Urinmeßgerät aus einem Meßbehälter 1 und einem auswechselbaren Urinbeutel 2. Über einen Schlauch 3 gelangt der Urin in die erste von beispielsweise vier Kammern 4 bis 7, die durch Zwischenwände 8, 9 und 10 gebildet werden. Ist die erste Kammer gefüllt, läuft der Urin über den oberen Rand 11 der Zwischenwand in die zweite Kammer 5 usw.

Nach Ablauf einer vorbestimmten Zeit wird die gesammelte Urinmenge an einer Skala 12 abgelesen. Anschließend wird ein Absperrorgan 13 geöffnet, und der Urin läuft aus den Kammern 4 und 5 direkt und aus den Kammern 6 und 7 über Kanäle 14 und 15 durch das Absperrorgan 13 und den Kanal 16 in den Urinbeutel 2.

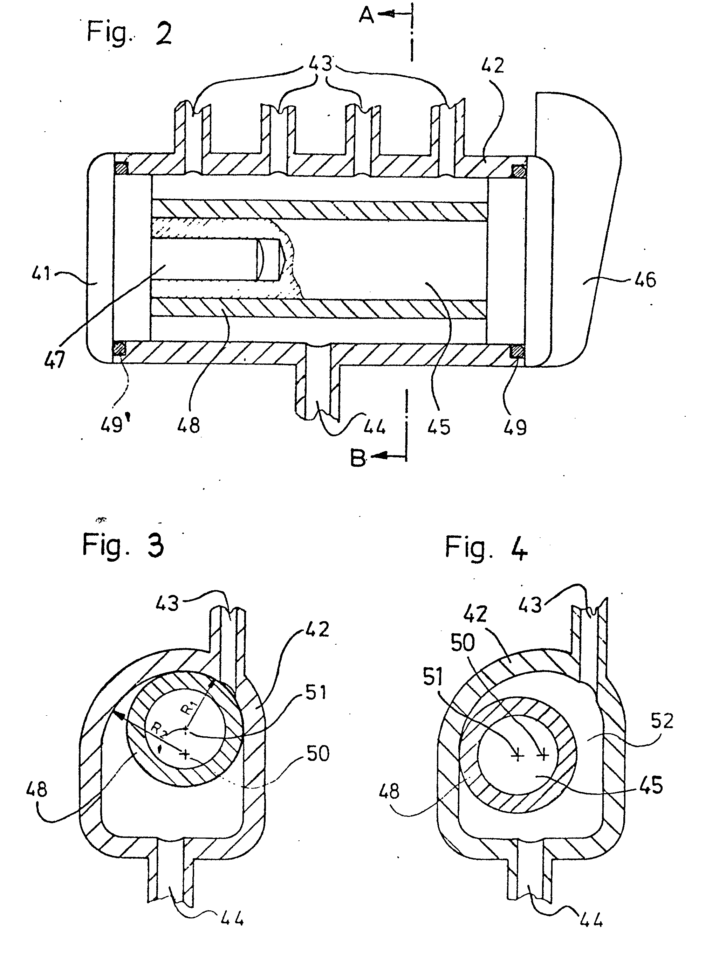

Gemäß Figur 2 besteht das Absperrorgan 13 aus einem Gehäuse 42 mit Einlaßöffnungen 43 und einer Auslaßöffnung 44, einem exzentrisch gelagerten zylindrischen Dichtelement 45 mit einer schlauchartigen Weichdichtung 48 und einem Drehknopf 46, einem Gegenlager 47 und zwei O-Ringen 49, 49' zur Abdichtung des Gehäuses 42 nach außen. Das Gehäuse 42 ist zu dem rotationssymmetrischen Dichtelement 45 überdimensioniert.

Figur 3 zeigt, daß sich das Dichtelement 45 um den Drehpunkt 50 dreht, wobei der Mittelpunkt 51 der Weichdichtung 48 vom geschlossenen zum geöffneten Zustand einen Kreisbogen in Pfeilrichtung beschreibt. Die Gehäuseinnenwand ist im Bereich der Einlaßöffnungen 43 mit einem Radius R 1 um den Mittelpunkt 51 des Dichtelementes 45 versehen, der zur Erzielung einer angemessenen Verpressung der Weichdichtung 48 um ein Geringes kleiner als der Radius der unbelasteten Weichdichtung 48 ist. Wenn das Dichtelement 45 zum Öffnen des Absperrorganes in Pfeilrichtung gedreht wird, beschreibt der am weitesten vom Drehpunkt 50 entfernt liegende Punkt der Weichdichtung 48 einen Bogen mit dem Radius $R_2$. Die Gehäuseinnenwand hat in diesem Bereich einen Radius, der größer als $R_2$ ist, um die Leichtgängigkeit des Dichtelementes 45 zu gewährleisten.

Im geöffneten Zustand entsteht gemäß Figur 4 zwischen der Weichdichtung 48 und der Gehäuseinnenwand ein Spalt 52, durch den der Urin von den Einlaßöffnungen 43 zur Auslaßöffnung 44 gelangt.

## Patentansprüche

1. Absperrorgan für den Auslaß eines Meßbehälters, insbesondere Urinmeßbehälters (1), mit einem auf seinem Außenumfang gummielastisch nachgiebigen Dichtelement, das in einem mit Ein- und Auslaßöffnungen (43; 44) versehenen Gehäuse (42) drehbar gelagert ist, dadurch gekennzeichnet, daß das Gehäuse (42) mehrere auf einer achsparallelen Linie nebeneinanderliegende Einlaßöffnungen (43) aufweist, die jeweils mit einer von mehreren Kammern (4, 5, 6, 7) des Meßbehälters (1) in Verbindung stehen, und daß die Dichtflächenabschnitte auf dem Dichtelement (45) nebeneinander auf einer achsparallelen Linie angeordnet sind.

2. Absperrorgan nach Anspruch 1, dadurch gekennzeichnet, daß das Dichtelement (45) rotationssymmetrisch gestaltet und in dem Gehäuse (42) exzentrisch drehbar gelagert ist.

3. Absperrorgan nach Anspruch 2, dadurch gekennzeichnet, daß die Innenwand des Gehäuses (42) im Bereich der Einlaßöffnungen (43) zu dem Mittelpunkt (51) des Dichtelementes (45) einen Radius ($R_1$) aufweist, der um ein geringes kleiner als der Radius der unbelasteten Oberfläche (48) des Dichtelementes (45) ist.

## Claims

1. Shutoff element for the outlet of a measuring container in particular of an urine measuring container (1) comprising a sealing element rubber-elastically flexible at its outer periphery which is pivotable in a housing (42) having inlet and outlet openings (43; 44) characterized in that the housing (42) includes a plurality of consecutively arranged axially aligned inlet openings (43) which communicate each with one of several chambers (4, 5, 6, 7) of the measuring container (1) and that the sealing surface portions on the sealing element (45) are consecutively arranged on an axially aligned line.

2. Shutoff element according to claim 1, characterized in that the sealing element (45) is of an axially symmetrical design and eccentrically pivotable in the housing (42).

3. Shutoff element according to claim 2, characterized in that the inner wall of the housing (42), in the area of the inlet openings (43), is provided with a radius ($R_1$) around the center point (51) of the sealing element (45), which is slightly smaller than the radius of the unloaded surface (48) of the sealing element (45).

## Revendications

1. Organe obturateur de la sortie d'un récipient de mesure, notamment de mesure d'urine (1), comportant un élément d'étanchéité flexible ayant l'élasticité du caoutchouc sur sa périphérie extérieure et qui est monté à rotation dans un boîtier (42) pourvu d'orifices d'admission et de décharge (43; 44), caractérisé en ce que le boîtier (42) comporte plusieurs orifices d'admission (43) juxtaposés sur une ligne parallèle à l'axe et dont chacun est relié à l'une de plusieurs chambres (4, 5, 6, 7) du récipient de mesure (1), et en ce que les parties de surfaces d'étanchéité sont disposées sur l'élément d'étanchéité (45) en juxtaposition sur une ligne parallèle à l'axe.

2. Organe obturateur selon la revendication 1, caractérisé en ce que l'élément d'étanchéité (45) est agencé de manière à présenter une symétrie de révolution et est monté à rotation et excentriquement dans le boîtier (42).

3. Organe obturateur selon la revendication 2, caractérisé en ce que la paroi intérieure du boîtier (42) présente, dans la zone des orifices d'admission (43) et à partir du centre (51) de l'élément d'étanchéité (45), un rayon (R1) légèrement inférieur au rayon de la surface non sollicitée (48) de l'élément d'étanchéité (45).

# Fig. 1

Fig. 2

Fig. 3

Fig. 4